# EUROPEAN PATENT SPECIFICATION

(11) **EP 2 535 408 B1**
(45) Date of publication and mention of the grant of the patent: **12.11.2014**
(21) Application number: 11742001.8
(22) Date of filing: 01.02.2011
(51) Int. Cl.: C12N 15/09, C12M 1/00, C12M 1/34, G01N 35/08, G01N 37/00, C12Q 1/68, B01J 19/00

(54) **MICROCHIP FOR NUCLEIC ACID AMPLIFICATION REACTION AND AND PROCESS FOR PRODUCTION THEREOF**
MIKROCHIP FÜR NUKLEINSÄURE-AMPLIFIKATIONSREAKTIONEN SOWIE HERSTELLUNGSVERFAHREN DAFÜR
MICROPUCE POUR RÉACTION D'AMPLIFICATION D'ACIDE NUCLÉIQUE ET PROCÉDÉ DE PRODUCTION ASSOCIÉ

(30) Priority: 10.02.2010 JP 2010027200
(43) Date of publication of application: 19.12.2012
(73) Proprietor: Sony Corporation, Tokyo 108-0075 (JP)
(72) Inventor: WATANABE, Hidetoshi, Tokyo 108-0075 (JP); ABE, Tomoteru, Tokyo 108-0075 (JP); YOTORIYAMA, Tasuku, Tokyo 108-0075 (JP)
(74) Representative: Müller - Hoffmann & Partner
(86) International application number: PCT/JP2011/000543
(87) International publication number: WO 2011/099251

(56) References cited:
- WO-A1-01/07581
- WO-A1-96/00301
- WO-A1-2007/083693
- JP-A- 2007 043 998
- JP-A- 2008 304 356
- US-A- 5 599 660
- US-A- 5 922 591
- US-A- 5 958 698
- US-A1- 2003 087 300
- US-A1- 2005 208 539
- US-A1- 2007 259 348
- US-A1- 2008 241 890
- US-B1- 6 440 725
- GODDARD ET AL: "Polymer surface modification for the attachment of bioactive compounds", PROGRESS IN POLYMER SCIENCE, PERGAMON PRESS, OXFORD, GB, vol. 32, no. 7, 1 July 2007 (2007-07-01), pages 698-725, XP022133245, ISSN: 0079-6700, DOI: 10.1016/J.PROGPOLYMSCI.2007.04.002

## Description

### Technical Field

The present invention relates to a microchip for a nucleic acid amplification reaction and a method of manufacturing the same, more particularly to a microchip for a nucleic acid amplification reaction in which a plurality of reagents needed for the reaction are laminated and anchored in a prescribed order in wells configured to function as reaction sites of the nucleic acid amplification reaction, or the like.

### Background Art

In recent years, by applying a microfabrication technique in the semiconductor industry, microchips having wells and flow channels for performing chemical and biological analyses formed on a substrate made of silicon or glass (for example, see Patent Document 1) have been developed. These microchips have begun to be used, for example, for an electrochemical detector of liquid chromatography or a small-sized electrochemical sensor in a practical medical field.

Such analysis system using the microchips is referred to as µ-TAS (micro-Total-Analysis System), Lab-on-chip, biochip or the like, and attracts attention as a technology that can speed up, increase efficiency of, and integrate the chemical and biological analyses, or decrease a size of analysis equipment.

Since the µ-TAS can analyze a small amount of samples and the microchips can be disposable (single-use), it is expected to apply it to the biological analysis that handles, specifically, a trace amount of precious samples or many test bodies.

An example of the application of the µ-TAS is a photodetector which introduces substances into a plurality of areas provided in microchips, and optically detect the substances or their reaction products thereof. An example of the photodetector is a nucleic acid amplification apparatus (for example, a real time PCR apparatus) that proceeds nucleic acid amplification reaction in wells of microchips, and optically detect or quantify amplified nucleic acid strands.

Conventionally, a microchip type nucleic acid amplification apparatus adopts a method to perform the reaction, by mixing all reagents needed for a nucleic acid amplification reaction and template DNAs (target nucleic acid strands) in advance, and introduce the mixed liquid into a plurality of wells provided in microchips. Thus, it is laborious to mix all reagents needed and target nucleic acid strands in advance. In addition, it takes a certain period of time until the mixed liquid is introduced into the wells, so it has a problem that the reaction proceeds in the mixed liquid during the time period. Once the reaction proceeds before the entering of the mixed liquid into the wells is completed, a reaction time cannot be controlled strictly, which may be a factor that decreases the determination precision of the amplified nucleic acid strands.

In general, the PCR method adopts a method called a hot start method in order to strictly control the reaction time. The hot start method is a method to avoid non-specific amplification reaction caused by misannealing of an oligonucleotide primer, and to provide an intended amplified product. In the PCR method, the hot start method is achieved by heating a mixed liquid including reagents other than enzymes (in general, heat-resistant derived bacteria DNA polymerase) and target nucleic acid strands to denaturation temperature of the oligonucleotide primer, adding the enzymes only after reaching the denaturation temperature, and then performing a normal temperature cycle.

In relation to the present invention, Patent Document 2 discloses a micro fluid chip including an oligonucleotide primer needed for a nucleic acid amplification reaction, a substrate, an enzyme and other reagents in a solid state in flow channels. In the micro fluid chip, remaining reagents needed for the reaction in a liquid state are sent to the flow channels, the reagents in the liquid state and the reagents in the solid state are contacted, and the reagents in the solid state are dissolved to start the reaction. In the Patent Document 2, there is no description that the oligonucleotide primer, the substrate, the enzyme and other reagents are laminated and anchored in the flow channels.
Patent Document 1: Japanese Patent Application Laid-open No. 2004-219199
Patent Document 2: Japanese Patent Application Laid-open No. 2007-43998

US 6,440,725 B1 teaches a cartridge for separating a desired analyte from a fluid sample which includes a sample port and a sample flow path extending from the port through the body of the cartridge.

JP 2008 304356 A discloses a reagent sealing section on a lab-on-chip having a plurality of reaction tubs filled with the reagent, the reaction liquid is added to the reaction tubes and reaction is performed. The reagent is distributed and stored in the sealing layers or covered with the sealing layers. The sealing layers are heated together with the reaction liquid, thereby bringing the reagent into contact with the reaction liquid to form a reactable state.

WO 2007/083693 A1 concerns a pyrophosphoric acid sensor that makes use of a primer extension reaction in a method of measuring pyrophosphoric acid in SNP. There is provided a pyrophosphoric acid sensor composed of an insulating substrate, formed thereon an electrode group consisting of measuring electrode and counter electrode, and superimposed on the substrate multiple reaction reagent layers consisting of pyrophosphatase, glyceraldehyde-3-phosphate dehydrogenase, diaphorase, glyceraldehyde-3-phosphate, oxidized nicotinamide adenine dinucleotide, electron mediator, magnesium salt and buffer solution components wherein the reaction reagent layer containing buffer solution components is separated from the enzyme-containing reaction reagent layer. The reaction reagent layer containing glyceraldehyde-3-phosphate is separated from the reaction reagent layer containing buffer solution components.

US 5,599,660 relates to a method and preparation for the storage and delivery of purified reagents. In one aspect, the preparation comprises an amount of a first wax carrier, the first wax carrier having a first melting point, and an amount of a first reagent, wherein first reagent is a substantially purified preparation of at least one biological or chemical reagent. The first wax carrier and the first reagent are combined to form a solid first reagent portion. The solid mixture is combined with a second reagent portion comprised of at least one biological or chemical reagent in an inactive form.

### Disclosure of the Invention

### Problem to be solved by the Invention

As described above, in the conventional microchip type nucleic acid amplification apparatus, since the reagents etc. are mixed in advance and introduced into the wells, it is laborious, and it had the following problem. The reaction time cannot be control strictly, so the analytical precision decreases.

Therefore, a principal object of the present invention is to provide a microchip for a nucleic acid amplification reaction which allows high-precision analysis by a simple method.
This is achieved by the subject-matter of the independent claims.

### Means for solving the Problem

In order to solve the above-mentioned problem, the present invention provides a microchip for a nucleic acid amplification reaction, including an entrance through which a liquid enters from the outside; a plurality of wells configured to function as reaction sites of the nucleic acid amplification reaction; and a flow channel through which the liquid entered through the entrance is fed into each of the wells, in which a plurality of reagents needed for the reaction are laminated and anchored in a prescribed order in each of the wells.

In the microchip for the nucleic acid amplification reaction, an anchored layer of an oligonucleotide primer may be laminated over an anchored layer of an enzyme, or, in an opposite manner, an anchored layer of an enzyme may be laminated over an anchored layer of an oligonucleotide primer. Between the anchored layer of the enzyme and the anchored layer of the oligonucleotide primer, an anchored layer of a reaction buffer solute may be laminated.

Also, the present invention provides a method of manufacturing a microchip for a nucleic acid amplification reaction, including a first step of laminating and anchoring a plurality of reagents needed for the reaction in a prescribed order in each of a plurality of wells configured to function as reaction sites of the nucleic acid amplification reaction, formed on a substrate layer.

The method of manufacturing the microchip for the nucleic acid amplification reaction further includes a second step of activating and adhering surfaces of substrate layers which the reagents are laminated and anchored to, in which the first step includes processes of dropping and drying an enzyme solution in each of the wells, and then dropping and drying an oligonucleotide primer solution.

In the method of manufacturing the microchip for the nucleic acid amplification reaction, the first step favorably includes processes of dropping and drying a reaction buffer solute solution after the dropping and drying of the enzyme solution in each of the wells, and before the dropping of the oligonucleotide primer solution.

In addition, in the method of manufacturing the microchip for the nucleic acid amplification reaction, the second step may include process of activating the surfaces of the substrate layers with an oxygen plasma treatment or a vacuum ultraviolet light treatment.

In the present invention, "a nucleic acid amplification reaction" includes a conventional PCR (polymerase chain reaction) method performing a temperature cycle, and a variety of isothermal amplification methods involving no temperature cycle. Examples of the isothermal amplification methods include an LAMP (Loop-Mediated Isothermal Amplification) method, an SMAP (Smart Amplification Process) method, an NASBA (Nucleic Acid Sequence-Based Amplification) method, an ICAN (Isothermal and Chimeric primer-initiated Amplification of Nucleic acids) method (trademark), a TRC (transcription-reverse transcription concerted) method, an SDA (strand displacement amplification) method, a TMA (transcription-mediated amplification) method, a RCA (rolling circle amplification) method and the like. Besides, "the nucleic acid amplification reaction" widely includes nucleic acid amplification reactions for amplifying nucleic acids at an alternating temperature or at a constant temperature. Also, "the nucleic acid amplification reaction" includes the reactions involving quantification of amplified nucleic acid strands such as a real time PCR (RT-PCR) method and an RT-RAMP method.

In addition, "reagents" include the reagents needed for obtaining the amplified nucleic acid strands in the above-mentioned nucleic acid amplification reaction, and specifically include an oligonucleotide primer having a complementary base sequence to target nucleic acid strands, a nucleic acid monomer (dNTP), an enzyme, a reaction buffer solution (buffer) solute and the like.

### Effect of the Invention

The present invention provides a microchip for a nucleic acid amplification reaction which allows high-precision analysis by a simple method.

### Brief Description of Drawings

[Fig. 1] A schematic top view of a microchip for a nucleic acid amplification reaction according to the present invention.
[Fig. 2] A schematic cross-sectional view of the microchip (Fig. 1, p-p cross-section).
[Figs. 3] Schematic views illustrating anchored layers of reagents laminated in each of the wells of the microchip.
[Fig. 4] A schematic view illustrating an alternative example of anchored layers of reagents laminated in a well of a microchip.
[Fig. 5] A flowchart illustrating a method of manufacturing a microchip for a nucleic acid amplification reaction according to the present invention.
[Fig. 6] A schematic view illustrating a method of laminating anchored layers of reagents in a well.

### Best Modes for Carrying Out the Invention

Hereinafter, preferred embodiments for carrying out the present invention will be described with reference to the drawings. The embodiments described below illustrate only examples of typical embodiments of the present invention, and the scope of the present invention is not narrowly interpreted by the embodiments. The embodiments will be described in the following order.
1. A microchip for a nucleic acid amplification reaction
2. A method of manufacturing a microchip for a nucleic acid amplification reaction

### (2-1) Formation of a substrate layer a1

### (2-2) Anchoring of an enzyme and a primer to wells

### (2-3) Surface activation and adhesion of substrate layers a1 and a2

### 1. A microchip for a nucleic acid amplification reaction

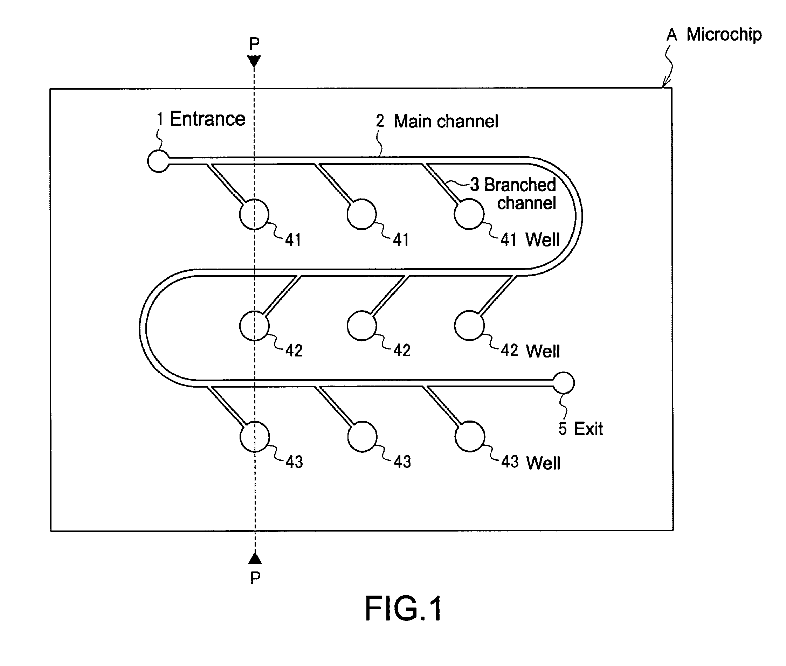

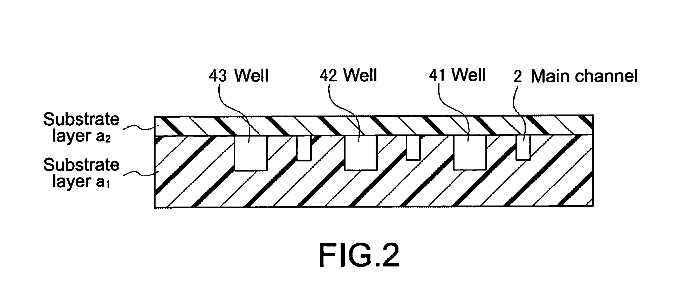

A schematic top view of a microchip for a nucleic acid amplification reaction according to the present invention (hereinafter simply referred to as a "microchip") is shown in Fig. 1, and a schematic cross-sectional view thereof is shown in Fig. 2. Fig. 2 corresponds to a p-p cross-section in Fig. 1.

A microchip designated as a symbol A includes an entrance 1 through which a sample solution enters from the outside, a plurality of wells configured to function as reaction sites of a nucleic acid amplification reaction, a main flow channel 2 that communicates with the entrance 1 at one end, and branched flow channels 3 branching from the main flow channel 2. The other end of the main flow channel 2 is configured to be an exit 5 that drains the sample solution to the outside. The branched flow channels 3 branch from the main flow channel 2 at positions between a communication part to the entrance 1 of the main flow channel 2 and a communication part to the exit 5, and are connected to respective wells. The sample solution can contain DNA, genome RNA, mRNA or the like that functions as a template (a target nucleic acid strand) in the nucleic acid amplification reaction.

Herein, the case that a total of nine wells are provided at equal intervals in three rows and three columns in the microchip A is given as an example. These nine wells are zoned in three sections. In Fig. 1., the three wells in the upper section are designated by a symbol 41, the three wells in the middle section are designated by a symbol 42, and the three wells in the lower section are designated by a symbol 43. The sample solution entered from the entrance 1 is sent toward the exit 5 through the main flow channel 2, and is sequentially fed to the internal from the branched flow channels 3 and the wells provided upstream in a solution sending direction. Alternatively, in the microchip A, the exit 5 may not be an essential component, the microchip A may be configured so that the sample solution entered from the entrance 1 is not discharged to the outside.

The microchip A is constructed by adhering a substrate layer a2 to a substrate layer a1, on which the entrance 1, the main flow channel 2, the branched flow channel 3, the wells 41, 42, and 43, and the exit 5 are formed. The material of the substrate layer a1 or a2 can be glass and a variety of plastics (polypropylene, polycarbonate, cyclo olefin polymer, polydimethyl siloxane). When the nucleic acid strands amplified in the wells 41, 42 and 43 are optically detected or quantified, the materials of the substrate layer a1 or a2 are preferably selected from materials having light permeability, less autofluorescence, and less optical errors because of a small wavelength dispersion.

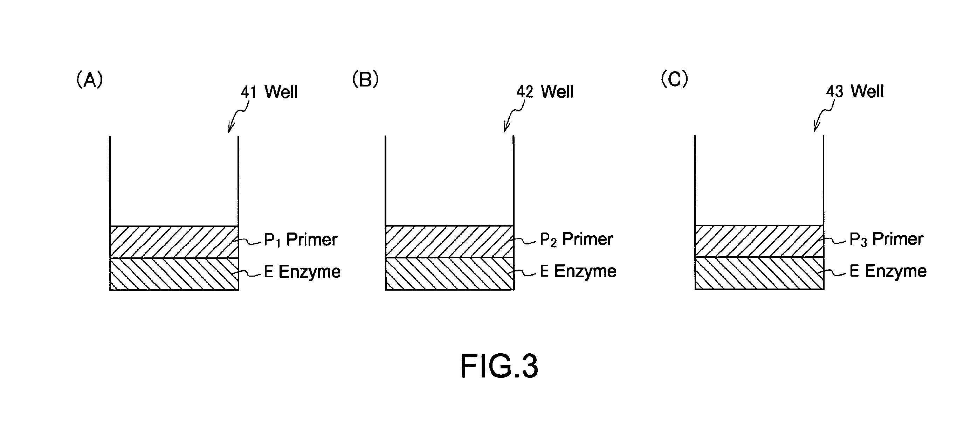

A plurality of reagents needed for the nucleic acid amplification reaction are laminated and anchored in a prescribed order in the wells 41, 42 and 43. Figs. 3 show examples of anchored layers of the reagents laminated in each of the wells. Fig. 3(A), (B) and (C) show the anchored layers of the reagents laminated in the well 41, the well 42 and the well 43, respectively.

The reagents anchored to the wells are the reagents needed for obtaining the amplified nucleic acid strands in the nucleic acid amplification reaction, and specifically include an oligonucleotide primer having a complementary base sequence to target nucleic acid strands, a nucleic monomer (dNTP), an enzyme, a reaction buffer solution (buffer) solute and the like. The anchoring reagents can be one or more than one of these.

The order to laminate these reagents can be in any order such as the order of "oligonucleotide primer, dNTP, enzyme, buffer solute" or the order of "buffer solute, enzyme, dNTP, oligonucleotide primer".

In addition, a part of these reagents, for example, the primer and the buffer solute, or the primer and dNTP may be mixed and anchored.

The reagents needed for detecting and quantifying the amplified nucleic acid strands, such as a fluorescent reagent (fluorescent pigment) or a phosphorescent reagent (phosphorescent pigment), can be anchored in the wells as needed, although it is not essential for obtaining the amplified nucleic acid strands.

Here, a case is shown that first the anchored layers of the enzymes E are formed in the wells 41, 42 and 43, and then the anchored layers of the oligonucleotide primers (hereinafter simply referred to as "primers") P1, P2 and P3 are laminated thereover. As described later, an anchored layer of a reaction buffer solute may be laminated between the anchored layer of the enzyme and the anchored layer of the primer (the details are described below).

The primers P1, P2 and P3 may be the primers having the same base sequences, but may be the primers having different base sequences when a plurality of target nucleic acid strands are amplified in the microchip A. For example, when a genotype is determined using the microchip A, the primers having different base sequences corresponding to the base sequences of respective genotypes are anchored to the wells 41, 42 and 43, respectively. When a contagium is determined using the microchip A, the primers having different base sequences corresponding to the gene sequences of respective viruses and microbes are anchored similarly. In this regard, the enzymes E anchored to the respective wells need to be the same.

Thus, the reagents needed for the reaction are anchored to the well in advance, so that the reaction can be started only by feeding the remaining reagents and the sample solution containing the target nucleic acid strands into each of the wells from the entrance 1, in the microchip A. This makes it possible to decrease the number of the reagents mixed in advance, and to avoid the labor of mixing, which realizes simple analysis.

Further, all of the reagents needed to the reaction are anchored to the well in advance, so that the reaction can be started only by feeding the sample solution containing the target nucleic acid strands alone, which realizes more simple analysis.

In addition, in the microchip A, the reaction is started only after the remaining regents and the sample solution containing the target nucleic acid strands are fed into the respective wells and the reagents anchored to the wells are dissolved, so that the reaction time can be controlled strictly and high-precision analysis can be provided.

Furthermore, the anchored layer of the primer that is added to the reaction system in an excess amount and is relatively stable to the change in temperature etc. is laminated over the anchored layer of the enzyme that is unstable to change in temperature, change in humidity, and light and easily decreases its activity or be deactivated as compared with the primer, dNTP and the buffer solute, so that the anchored layer of the primer can protect the anchored layer of the enzyme. Thus, the decrease in the activity or the deactivation of the enzyme caused by the change in temperature etc. at the time of manufacture and storage of the microchip A can be prevented.

Although the above description is explained by the case that a total of nine wells are provided in three rows and three columns at equal intervals in the microchip is given as an example, any numbers and positions of the wells can be used, and the shapes of the wells are not limited to the cylinder shown in figures. In addition, the configuration of the flow channels to feed the sample solution entered from the entrance 1 into the respective wells is not limited to the aspect of the main flow channel 2 and the branched flow channel 3 shown in the figure. Further, although it is explained that the entrance 1 or the like is formed on the substrate layer a1, the entrance 1 or the like may be each formed both on the substrate layer a1 and the substrate layer a2. The substrate layers constituting the microchip may be two or more.

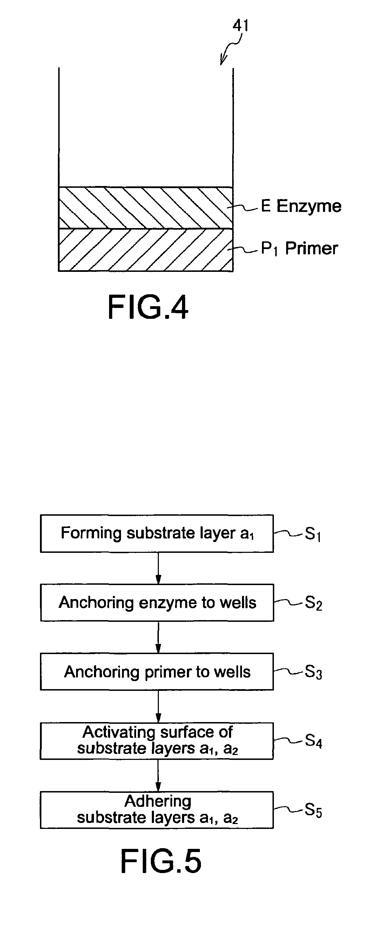

In addition, although the above description is explained by the case that the anchored layer of the primer is laminated over the anchored layer of the enzyme, the reagents may be laminated in any order, e.g., the anchored layer of the enzyme may be laminated over the anchored layer of the primer (see Fig. 4). In this case, an anchored layer of a reaction buffer solute may be laminated between the anchored layer of the enzyme and the anchored layer of the primer (the details are described below).

When the anchored layer of the primer is laminated atop, after the dissolving of the anchored layer of the primer with the sample solution fed into the respective wells from the entrance 1 and before dissolving of the enzyme to start the reaction , the primer, that is dissolved beforehand, may be interdiffused (cross-contaminated) between the wells. In contrast, when the anchored layer of the enzyme is laminated atop, cross-contamination of the primer can be prevented because the reaction starts after the dissolving of the anchored layer of the enzyme, and as soon as the dissolving of the anchored layer of the primer is started to be.

### 2. A method of manufacturing a microchip for a nucleic acid amplification reaction

Next, the method of manufacturing the microchip according to the present invention will be described referring to the flowchart in Fig. 5. As an example, it will be explained below taking the above-mentioned microchip A.

### (2-1) Formation of a substrate layer a1

In Fig. 5, a symbol S1 is a step of forming a substrate layer a1. In this step, an entrance 1, a main flow channel 2, a branched flow channel 3, wells 41, 42 and 43 and an exit 5 are formed on the substrate layer a1. The formation of the entrance 1 and the other components on the substrate layer a1 can be carried out by, for example, wet-etching or dry-etching of a glass substrate layer, or by nanoimprinting, injection molding or cutting work of a plastic substrate layer.

### (2-2) Anchoring of an enzyme and a primer to wells

A symbol S2 is a step of anchoring the enzyme to the wells. A symbol S3 is a step of anchoring the primer to the wells. The steps S2 and S3 correspond to the step of laminating and anchoring a plurality of reagents needed for the reaction in a prescribed order in the wells (the first step).

The reagents anchored in the wells are the reagents needed to provide the amplified nucleic acid strands in the nucleic acid amplification reaction, and specifically include an oligonucleotide primer having a complementary base sequence to target nucleic acid strands, a nucleic acid monomer (dNTP), an enzyme, a reaction buffer solution (buffer) solute and the like. The anchoring reagents can be one or more than one of these.

The order to laminate these reagents can be in any order such as the order of "primer, dNTP, enzyme, buffer solute" or the order of "buffer solute, enzyme, dNTP, primer".

In addition, a part of these reagents, for example, the primer and the buffer solute, or the primer and dNTP may be mixed and anchored.

Here, the solution of the enzyme E is dropped and dried in the wells 41, 42 and 43 in the step S2, and the solutions of the primers P1, P2 and P3 are dropped and dried respectively in the step S3, whereby the anchored layer of each primer is laminated over the anchored layer of the enzyme. The primers P1, P2 and P3 may be the primers having the same base sequences, but may be the primers having different base sequences when a plurality of target nucleic acid strands are amplified in the microchip A. In this regard, the enzymes E anchored to the respective wells need to be the same.

The anchoring of the reagents are carried out by, for example, air drying, vacuum drying, or freeze drying the dropped solution, and preferably by gradual drying. As to the enzyme, critical point drying is also effective in order to prevent the decrease in the activity or the deactivation.

At that time, the enzyme anchored in the step S2 may be redissolved by the primer solution dropped in the step S3. When the enzyme and the primer are mixed due to redissolving, it is unfavorable because the amplifying of primer dimers may occur.

In order to prevent the mixing of the enzyme and the primer, in the step S3, it is preferable to keep the microchip at low temperature (about -10°C) in advance, so that the primer solution dropped may be frozen and freeze dried.

Alternatively, the following method may be used. The method is, keeping microchip at low temperature in advance, dropping water and freezing it, then dropping the primer solution and conducting its freeze drying, and then air drying to evaporate an intermediate layer of ice.

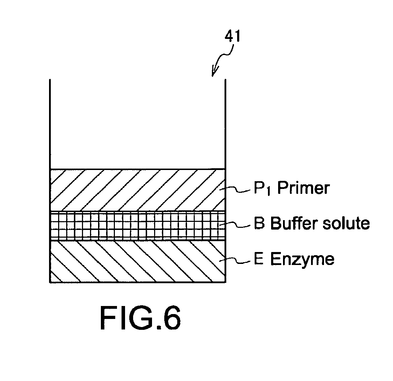

In addition, it is more preferable to use the following method. The method is, keeping the microchip at low temperature in advance, dropping the buffer solute solution and conducting its freeze drying, laminating an anchored layer of a buffer solute B over the anchored layer of the enzyme E, and after that, dropping the primer solution, then air drying, vacuum drying or freeze drying. Thus, a three-layered structure, where the anchored layer of the buffer solute is laminated between the anchored layer of the enzyme and the anchored layer of the primer, is formed in each of the well (see Fig. 6).

Also, these methods can be employed in order to prevent the mixing of the primer, when the anchored layer of the enzyme is laminated over the anchored layer of the primer.

### (2-3) Surface activation and adhesion of substrate layers a1 and a2

A symbol S4 is a step of activating the surfaces of the substrates layers a1 and a2. A symbol S5 is a step of adhering the substrate layers a1 and a2. The steps S4 and S5 correspond to the step of activating and adhering the surfaces of the substrate layers to which the reagents are laminated and anchored (the second step).

The substrate layer a1 and the substrate layer a2 can be adhered by, for example, adhesion using an adhesive or an adhesive sheet, heat seal, anodic bonding or ultrasonic bonding.

Also, there can be used a method of activating and adhering the surfaces of the substrate layers with an oxygen plasma treatment or a vacuum ultraviolet light treatment. Plastics such as polydimethyl siloxane and glass have high affinity. When their surfaces are activated and contacted, dangling bonds are reacted to form strong covalent bonds, i.e., Si-O-Si silanol bonds, thereby providing the adhesion having sufficient strength. The oxygen plasma treatment or the vacuum ultraviolet light treatment is carried out by setting appropriate conditions depending on the materials of the substrate layers.

Thus, by separately anchoring and laminating the enzyme and the primer in the wells of the substrate layers on which the wells are formed, the reagents can be anchored without promoting the amplification reaction of the primer dimers in the course of dropping and drying the solution, unlike in the case of mixing the enzyme and the primer and anchoring them.

Furthermore, the anchored layer of the primer that is added to the reaction system in an excess amount and is relatively stable to the change in temperature etc. is laminated over the anchored layer of the enzyme that is unstable to change in temperature, change in humidity and light and easily decreases its activity or be deactivated, so that the anchored layer of the enzyme can be protected from heat, plasma or ultraviolet light irradiation in the step S4. In other words, when the surfaces of the substrate layers to which the reagents are anchored are activated by the oxygen plasma treatment or the vacuum ultraviolet light treatment, the anchored layer of the primer protects the enzyme beneath as well, whereby the decrease in the activity or the deactivation of the enzyme caused by the plasma or the ultraviolet light irradiation can be prevented.

Although the above description is explained by the case that the anchored layer of the primer is laminated over the anchored layer of the enzyme, the reagents may be laminated in any order, e.g., the anchored layer of the enzyme may be laminated over the anchored layer of the primer (see Fig. 4). In this case, it is also effective that an anchored layer of a reaction buffer solute is laminated between the anchored layer of the enzyme and the anchored layer of the primer.

### Industrial Applicability

The microchip for the nucleic acid amplification reaction according to the present invention allows high-precision analysis by a simple method. Thus, the microchip for the nucleic acid amplification reaction according to the present invention can be used for the microchip type nucleic acid amplification apparatus for genotype determination , contagium determination or the like. Description of Symbols

- A: microchip for nucleic acid amplification reaction
- E: enzyme
- P1, P2, P3: primer
- 1: entrance
- 2: main flow channel
- 3: branched flow channel
- 41, 42, 43: well
- 5: exit

## Claims

1. A microchip for a nucleic acid amplification reaction, comprising:
an entrance through which a liquid enters from the outside;
a plurality of wells configured to function as reaction sites of the nucleic acid amplification reaction; and
a flow channel through which the liquid entered through the entrance is fed into each of the wells,
wherein a plurality of reagents needed for the reaction are laminated and anchored in a prescribed order in each of the wells,
wherein an anchored layer of an oligonucleotide primer is laminated over an anchored layer of an enzyme.

2. The microchip for the nucleic acid amplification reaction according to claim 1,
wherein an anchored layer of a reaction buffer solute is laminated between the anchored layer of the enzyme and the anchored layer of the oligonucleotide primer.

3. A method of manufacturing a microchip for a nucleic acid amplification reaction, comprising:
a first step of laminating and anchoring a plurality of reagents needed for the reaction in a prescribed order in each of a plurality of wells configured to function as reaction sites of the nucleic acid amplification reaction, formed on a substrate layer, wherein the first step includes processes of dropping and drying an enzyme solution in each of the wells, and then dropping and drying an oligonucleotide primer solution,
further comprising:
a second step of activating and adhering surfaces of the substrate layers to which the reagents are laminated and anchored.

4. The method of manufacturing the microchip for the nucleic acid amplification reaction according to claim 3,
wherein the first step includes processes of dropping and drying a reaction buffer solute solution after the dropping and drying of the enzyme solution in each of the wells, and before the dropping of the oligonucleotide primer solution.

5. The method of manufacturing the microchip for the nucleic acid amplification reaction according to claim 4,
wherein the second step includes process of activating the surfaces of the substrate layers with an oxygen plasma treatment or a vacuum ultraviolet light treatment.

## Patentansprüche

1. Mikrochip für eine Nukleinsäureamplifikationsreaktion, umfassend:
einen Eingang, durch den eine Flüssigkeit von außen eintritt;
mehrere Vertiefungen mit einer Konfiguration, um als Reaktionsorte für die Nukleinsäureamplifikationsreaktion zu fungieren; und
einen Durchflusskanal, über den die durch den Eingang eingetretene Flüssigkeit jeder der Vertiefungen zugeführt wird,
wobei mehrere für die Reaktion benötigte Reagentien in einer vorgeschriebenen Reihenfolge in jeder der Vertiefungen laminiert und verankert sind,
wobei eine verankerte Schicht eines Oligonukleotid-Primers über eine verankerte Schicht eines Enzyms laminiert ist.

2. Mikrochip für die Nukleinsäureamplifikationsreaktion nach Anspruch 1,
wobei eine verankerte Schicht einer löslichen Reaktionspuffersubstanz zwischen der verankerten Schicht des Enzyms und der verankerten Schicht des Oligonukleotid-Primers laminiert ist.

3. Verfahren zur Herstellung eines Mikrochip für eine Nukleinsäureamplifikationsreaktion, bei dem man:
in einem ersten Schritt mehrere für die Reaktion benötigte Reagentien in einer vorgeschriebenen Reihenfolge in jeder von mehreren Vertiefungen mit einer Konfiguration, um als Reaktionsorte für die Nukleinsäureamplifikationsreaktion zu fungieren, gebildet auf einer Substratschicht, laminiert und verankert, wobei der erste Schritt Vorgänge beinhaltet, bei denen eine Enzymlösung in jeder der Vertiefungen abgesetzt und getrocknet wird und anschließend eine Oligonukleotid-Primer-Lösung abgesetzt und getrocknet wird,
wobei man ferner:
in einem zweiten Schritt Oberflächen der Substratschichten, an denen die Reagentien laminiert und verankert sind, aktiviert und bindet.

4. Verfahren zur Herstellung des Mikrochip für die Nukleinsäureamplifikationsreaktion nach Anspruch 3,
wobei der erste Schritt Vorgänge beinhaltet, bei denen eine Lösung einer löslichen Reaktionspuffersubstanz nach dem Absetzen und Trocknen der Enzymlösung in jeder der Vertiefungen und vor dem Absetzen der Oligonukleotid-Primer-Lösung abgesetzt und getrocknet wird.

5. Verfahren zur Herstellung des Mikrochip für die Nukleinsäureamplifikationsreaktion nach Anspruch 4,
wobei der zweite Schritt einen Vorgang beinhaltet, bei dem die Oberflächen der Substratschichten mit einer Sauerstoff-Plasma-Behandlung oder einer Behandlung mit ultraviolettem Licht im Vakuum aktiviert werden.

## Revendications

1. Puce pour une réaction d'amplification d'acide nucléique, comprenant :
une entrée par laquelle un liquide entre depuis l'extérieur ;
une pluralité de puits configurés pour fonctionner en tant que sites de réaction de la réaction d'amplification d'acide nucléique ; et
un canal d'écoulement par lequel le liquide entré par l'entrée est alimenté dans chacun des puits,
dans laquelle une pluralité de réactifs nécessaires pour la réaction sont laminés et ancrés dans un ordre prescrit dans chacun des puits,
dans laquelle une couche ancrée d'une amorce oligonucléotidique est laminée au-dessus d'une couche ancrée d'une enzyme.

2. Puce pour la réaction d'amplification d'acide nucléique selon la revendication 1,
dans laquelle une couche ancrée d'une soluté de tampon de réaction est laminée entre la couche ancrée de l'enzyme et la couche ancrée de l'amorce oligonucléotidique.

3. Procédé de fabrication d'une puce pour une réaction d'amplification d'acide nucléique, comprenant :
une première étape de laminage et d'ancrage d'une pluralité de réactifs nécessaires pour la réaction dans un ordre prescrit dans chacun d'une pluralité de puits configurés pour fonctionner en tant que sites de réaction de la réaction d'amplification d'acide nucléique, formés sur une couche de substrat, dans lequel la première étape comprend des processus de dépôt et de séchage d'une solution d'enzyme dans chacun des puits, et ensuite dépôt et séchage d'une solution d'amorce oligonucléotidique,
comprenant en outre :
une deuxième étape d'activation et d'adhésion aux surfaces des couches de substrat sur lesquelles les réactifs sont laminés et ancrés.

4. Procédé de fabrication de la puce pour la réaction d'amplification d'acide nucléique selon la revendication 3,
dans lequel la première étape comprend des processus de dépôt et séchage d'un soluté de tampon de réaction après le dépôt et le séchage de la solution d'enzyme dans chacun des puits, et avant le dépôt de la solution d'amorce oligonucléotidique.

5. Procédé de fabrication de la puce pour la réaction d'amplification d'acide nucléique selon la revendication 4,
dans lequel la deuxième étape comprend un processus d'activation des surfaces des couches de substrat avec un traitement par plasma à oxygène ou un traitement par lumière ultraviolette sous vide.
